# EUROPEAN PATENT APPLICATION

(11) **EP 1 908 845 A1**
(43) Date of publication of application: **09.04.2008**
(21) Application number: 06768311.0
(22) Date of filing: 19.07.2006
(51) Int. Cl.: C12P 13/02, C12P 17/10

(54) **METHOD FOR PRODUCING OPTICALLY ACTIVE 2-(N-SUBSTITUTED AMINOMETHYL)-3-HYDROXYBUTYRIC ACID ESTER**

(30) Priority: 20.07.2005 JP 2005210371
(71) Applicant: Kaneka Corporation, Kita-ku Osaka-shi, Osaka 530-8288 (JP)
(72) Inventor: YASOHARA, Yoshihiko, Takasago-shi, Hyogo 676-8688 (JP); YANO, Miho, Takasago-shi, Hyogo 676-8688 (JP); KAWANO, Shigeru, Takasago-shi, Hyogo 676-8688 (JP); KIZAKI, Noriyuki, Takasago-shi, Hyogo 676-8688 (JP)
(74) Representative: Vossius & Partner
(86) International application number: PCT/JP2006/314285
(87) International publication number: WO 2007/010944

(57) **Abstract**

The present invention relates to a method for producing optically active 2-(N-substituted aminomethyl)-3-hydroxybutyric acid esters wherein a 2-(N-substituted aminomethyl)-3-oxobutyric acid ester is treated with an enzyme source capable of stereoselectively reducing said ester to the corresponding optically active 2-(N-substituted aminomethyl)-3-hydroxybutyric acid ester having the (2S,3R) configuration. The present invention provides an efficient method for industrially producing optically active 2-(N-substituted aminomethyl)-3-hydroxybutyric acid esters, in particular such compounds having the (2S,3R) configuration, which are useful as intermediates for the production of medicinal compounds, among others.

## Description

### TECHNICAL FIELD

The present invention relates to a method for producing optically active 2-(N-substituted aminomethyl)-3-hydroxybutyric acid esters. Those compounds are useful, for example, as raw materials or intermediates for the synthesis of medicinal compounds required to be optically active.

### BACKGROUND ART

Optically active 2-(N-substituted aminomethyl)-3-hydroxybutyric acid esters, in particular such compounds having the (2S,3R) configuration, are compounds important as intermediates for the synthesis of β-lactam antibiotics, typically thienamycin. A method known for the production of those compounds comprises stereospecifically and catalytically reducing the carbonyl group in position 3 of 2-(N-substituted aminomethyl)-3-oxobutyric acid esters by the hydrogenation reaction using a ruthenium-optically active phosphine complex (Non-Patent Document 1; Patent Document 1). However, this catalytic reduction method requires the use of a very expensive optically active phosphine ligand for attaining a high level of stereoselectivity and the use of a high hydrogen pressure of about 1 to 10 MPa; for these and other reasons, this method is not fully satisfactory from the commercial production and economical viewpoint.

On the other hand, there are reports about the reduction reaction of the above-mentioned esters using an enzyme or microorganism as a catalyst. Thus, when ethyl 2-benzamidomethyl-3-hydroxybutyrate is reduced using baker's yeast, a mixture of (2S,3S) and (2R,3S) forms is obtained
(Patent Document 2). When ethyl 2-benzamidomethyl-3-hydroxybutyrate is reduced using microbial cells, mixtures of (2R,3S) and (2S,3S) forms in varying mixing ratio are obtained according to the microbial species used (Non-Patent Document 2). Further, when ethyl 2-phthaloylaminomethyl-3-oxobutyrate was reduced using a *Kluyveromyces marxianus*-derived reductase, a compound having the (2S,3R) configuration was detected (Patent Document 3;
Non-Patent Document 3).
Patent Document 1: Japanese Kokai Publication Hei02-134349
Patent Document 2: Japanese Kokai Publication Sho63-297360
Patent Document 3: United States Patent Application Publication 2003/0139464
Non-Patent Document 1:R. Noyori et al., "Stereoselective hydrogenation via dynamic kinetic resolution", J. Am. Chem. Soc., 111, 9134 (1989)
Non-Patent Document 2: Claudio Fuganti et al., "Microbial Generation of (2R,3S)- and (2S,3S)-Ethyl 2-Benzamidomethyl-3-hydroxybutyrate, a key intermediate in the synthesis of (3S,1'R)-3-(1'-hydroxyethyl)azetidin-2-one", J. Chem, Soc. Perkin Trans., 1, (1993), 2247
Non-Patent Document 3: Joo Hwan Cha et al., "Stereochemical control in diastereoselective reduction of α-substituted-β-ketoesters using a reductase purified from Kluyveromyces marxianus", Biotechnol. Lett., 24, 1695 (2002)

### SUMMARY OF THE INVENTION

It is an object of the present invention to provide a method for industrially producing optically active 2-(N-substituted aminomethyl)-3-hydroxybutyric acid esters, in particular such compounds having the (2S,3R) configuration. These compounds are utilized as intermediates for the synthesis of β-lactam antibiotics, among others.

The present inventors made investigations in an attempt to accomplish the above object and, as a result, discovered enzyme sources capable of stereoselectively reducing the carbonyl group of 2-(N-substituted aminomethyl)-3-oxobutyric acid esters to convert the same to the corresponding 2-(N-substituted aminomethyl)-3-hydroxybutyric acid esters having the (2S,3R) configuration and have now completed the present invention.

Thus, the present invention relates to
a method for producing optically active 2- (N-substituted aminomethyl)-3-hydroxybutyric acid esters represented by the general formula (5): 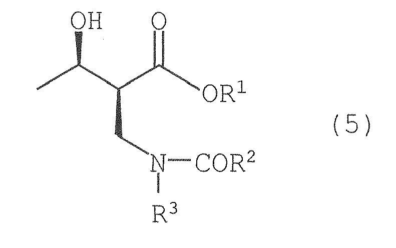 (wherein R¹ represents a lower alkyl group, which may optionally be substituted, an allyl group, an aryl group, which may optionally be substituted, or an aralkyl group, which may optionally be substituted and, as for R² and R³,
1) R³ is a hydrogen atom and R² represents a lower alkyl group, which may optionally be substituted, a lower alkoxy group, which may optionally be substituted, an aryl group, which may optionally be substituted, or an aralkyloxy group, which may optionally be substituted, or
2) R³ and -COR² together represent a phthaloyl group):,
   wherein a 2-(N-substituted aminomethyl)-3-oxobutyric acid ester represented by the general formula (6): 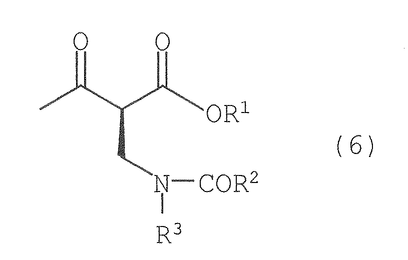 (wherein R¹, R² and R³ are as defined above): is treated with an enzyme source capable of stereoselectively reducing said ester to the corresponding optically active 3-hydroxybutyric acid ester having the (2S,3R) configuration.

### (Effect of the invention)

The present invention provides a method for industrially producing 2-(N-substituted aminomethyl)-3-hydroxybutyric acid esters having the (2S, 3R) configuration which are useful as intermediates for the production of medicinal compounds, among others.

### DETAILED DESCRIPTION OF THE INVENTION

In the following, typical modes of embodiment of the present invention are described in detail.

### 1. Substrate and product

The 2-(N-substituted aminomethyl)-3-oxobutyric acid ester as an example of the substrate to be used in the reduction reaction in accordance with the invention is a compound represented by the general formula (6):

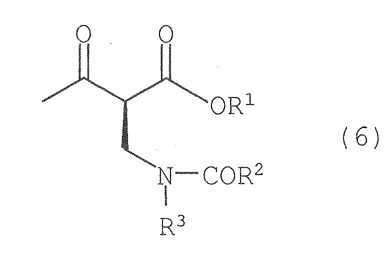

In the above formula, R¹ represents a lower alkyl group, which may optionally be substituted, an allyl group, an aryl group, which may optionally be substituted, or an aralkyl group, which may optionally be substituted and, as for R² and R³,
1) R³ is a hydrogen atom and R² represents a lower alkyl group, which may optionally be substituted, a lower alkoxy group, which may optionally be substituted, an aryl group, which may optionally be substituted, or an aralkyloxy group, which may optionally be substituted, or
2) R³ and -COR² together represent a phthaloyl group.

Thus, when R³ and R² make the combination 1) mentioned above, the compound represented by the above formula (6) is a compound represented by the formula (2): 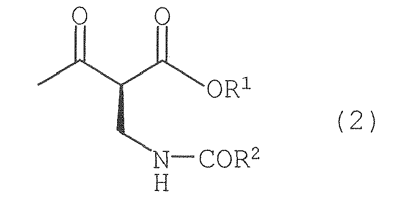 (wherein R¹ is as defined above and R² represents a lower alkyl group, which may optionally be substituted, a lower alkoxy group, which may optionally be substituted, an aryl group, which may optionally be substituted, or an aralkyloxy group, which may optionally be substituted):, and, when R³ and R² are related to each other in the manner mentioned above under 2), the compound represented by the formula (6) is a compound represented by the formula (4): 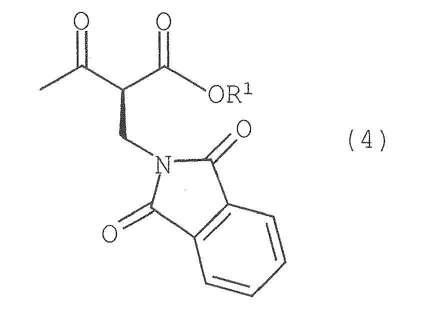 (wherein R¹ is as defined above).

Unless otherwise specified, the term "lower" denotes that the relevant group contains 1 to 7 carbon atoms, preferably 1 to 4 carbon atoms.

As the lower alkyl group, there may be mentioned, for example, a methyl group, an ethyl group, a chloromethyl group, an n-propyl group, an isopropyl group, an n-butyl group, an isobutyl group, a tert-butyl group, an n-pentyl group and a cyclohexyl group, among others. Preferred are a methyl group, an ethyl group, an n-propyl group, a butyl group and the like. These groups may be substituted. When the lower alkyl group is a substituted one, the substituent or substituents are not particularly restricted but each may be selected from among halogen atoms, a hydroxyl group, an amino group, a nitro group, a cyano group and so forth, provided that they will not adversely affect the reduction reaction in the practice of the present invention.

The aryl group, which may optionally be substituted, is not particularly restricted but includes, for example, a phenyl group, an o-methylphenyl group, an m-methylphenyl group, a p-methylphenyl group, an o-methoxyphenyl group, an m-methoxyphenyl group, a p-methoxyphenyl group, an o-fluorophenyl group, an m-fluorophenyl group, a p-fluorophenyl group, an o-chlorophenyl group, an m-chlorophenyl group, a p-chlorophenyl group, an o-nitrophenyl group, an m-nitrophenyl group, a p-nitrophenyl group, an o-trifluoromethylphenyl group, an m-trifluoromethylphenyl group, a p-trifluoromethylphenyl group, a naphthyl group, an anthracenyl group, a 2-furyl group, a 2-thiophenyl group and a 2-pyridyl group, among others. Preferred is a phenyl group, which may optionally be substituted; a phenyl group is more preferred.

The aralkyl group, which may optionally be substituted, is not particularly restricted but may be a benzyl group, a p-hydroxybenzyl group or a p-methoxybenzyl group, for instance.

The lower alkoxy group is not particularly restricted but includes a methyloxy group, an ethyloxy group, a chloromethyloxy group, an n-propyloxy group, an isopropyloxy group, an n-butyloxy group, an isobutyloxy group, a tert-butyloxy group, an n-pentyloxy group and a cyclohexyloxy group, among others. Preferred are a methyloxy group, an ethyloxy group, an n-propyloxy group and a butyloxy group, among others. These groups may be substituted and, as the substituent or substituents, there may be mentioned the same ones enumerated hereinabove referring to the alkyl group.

As the aralkyloxy group, which may optionally be substituted, there may be mentioned a benzyloxy group, a p-hydroxybenzyloxy group and a p-methoxybenzyloxy group, among others; a benzyloxy group is preferred, however.

Among those mentioned above, R¹ is preferably an alkyl group containing 1 to 4 carbon atoms, more preferably a methyl group. R² is preferably a phenyl group, which may optionally be substituted, more preferably a phenyl group, a p-nitrophenyl group or a p-chlorophenyl group, still more preferably a phenyl group. Also preferably, and -COR² together form a phthaloyl group. Further, the case where R¹ is a methyl group and R² is a phenyl group is particularly preferred.

In accordance with the present invention, the compound represented by the formula (6) given above is subjected to asymmetric reduction by treatment with an enzyme source capable of asymmetrically reducing said compound to give the corresponding optically active 2-(N-substituted aminomethyl)-3-hydroxybutylic acid ester represented by the general formula (5): 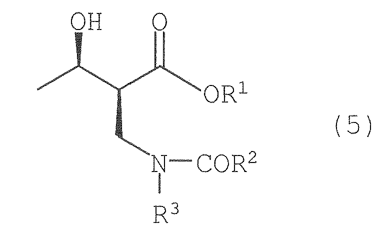 (wherein R¹, R² and R³ are as defined above).

It goes without saying that when a compound represented by the formula (2) or (4) given above is used as the compound represented by the formula (6) given above, the reduction product is a compound represented by the formula (1) or (3) given below, respectively.

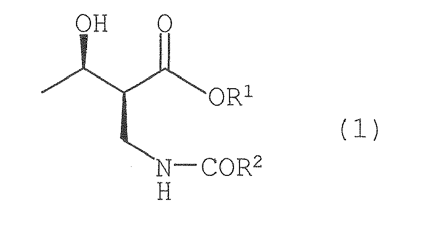

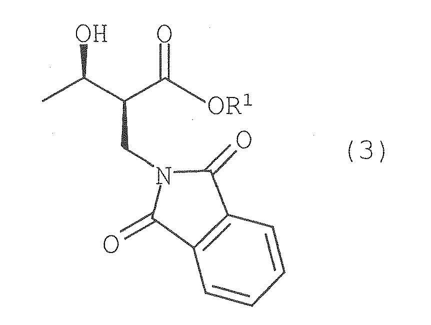

### 2. Enzyme source

Usable as the enzyme source in the practice of the invention is one derived from a microorganism capable of converting a 2-(N-substituted aminomethyl)-3-oxobutyric acid ester to the corresponding optically active 2-(N-substituted aminomethyl)-3-hydroxybutyric acid ester. The term "one derived from a microorganism" as used herein includes, within the meaning thereof, cells of the microorganism as such, a culture of the microorganism, a product obtained by subjecting cells of the microorganism to certain treatment, the enzyme obtained from the microorganism and, further, a transformant obtained by introduction of a DNA coding for the enzyme derived from the microorganism and having such reducing activity as mentioned above. These may be used singly or in combination of two or more species. These enzyme sources may be immobilized so that they may be used repeatedly.

### 3. Assaying of capability to cause conversion to 2-(N-substituted aminomethyl)-3-hydroxybutyric acid esters

The microorganism capable of converting 2- (N-substituted aminomethyl)-3-oxobutyric acid esters to optically active 2-(N-substituted aminomethyl)-3-hydroxybutyric acid esters can be found out by such a method as described below. Thus, for example, the following method is employed. A 5-ml portion of a liquid medium (pH 7) having a composition comprising 40 g of glucose, 3 g of yeast extract, 6. 5 g of diammonium hydrogen phosphate, 1 g of potassium dihydrogen phosphate, 0.8 g of magnesium sulfate heptahydrate, 60 mg of zinc sulfate heptahydrate, 90 mg of iron sulfate heptahydrate, 5 mg of copper sulfate pentahydrate, 10 mg of manganese sulfate tetrahydrate, 100 mg of sodium chloride (each per 1 liter) is placed in a test tube, sterilized and then aseptically inoculated with a microorganism, followed by 2 to 3 days of shake culture at 30°C. Thereafter, cells are collected by centrifugation, suspended in 0.5 to 5 ml of phosphate buffer containing 2 to 10% of glucose, added to 0.5 to 25 mg of methyl 2-benzamidomethyl-3-oxobutyrate and the like (belonging to the category of 2-(N-substituted aminomethyl)-3-oxobutyric acid esters) placed in advance in a test tube, and shake-cultured at 30°C for 2 to 3 days. On that occasion, the cells collected by centrifugation may also be used after drying in a desiccator or using acetone. Further, on the occasion of reacting the microorganism or a treatment product derived therefrom with the 2-benzamidemethyl-3-oxobutyl esters, NAD⁺ and/or NADP⁺ may be added, together with glucose dehydrogenase and glucose or with formate dehydrogenase and formic acid. An organic solvent may be caused to coexist in the reaction system. After the conversion reaction, the reaction mixture is extracted with an appropriate organic solvent, and the 2-benzamidomethyl-3-hydroxybutyl esters formed are assayed by high-performance liquid chromatography, for instance.

### 4. Microorganism

As for the microorganism which can be used in the practice of the present invention, any of those microorganisms capable of converting the 2-(N-substituted aminomethyl)-3-oxobutyric acid esters to the corresponding (2S,3R)-2-(N-substituted aminomethyl)-3-hydroxybutyric acid esters can be used. For example, there may be mentioned microorganisms belonging to the genera *Candida, Geotrichum, Galactomyces, Saccharomycopsis, Achromobacter, Arthrobacter, Bacillus, Brevundimonas, Xanthomonas, Devosia, Ralstonia, Lactobacillus, Lenconostoc, Microsporum* and *Moniliella,* among others.

More preferred are such species as *Candida kefyr, Candida oleophila, Candida maris, Geotrichum eriense, Galactomyces reessii, Saccharomycopsismalanga, Achromobacter xylosoxidans, Achromobacter denitrificans, Arthrobacter paraffineus, Arthrobacter nicotianae, Bacillus amylolyticus, Bacillus circulans, Bacillus cereus, Bacillus badius, Bacillus sphaericus, Brevundimonas diminuta, Xanthomonas* sp., *Devosia riboflavina, Ralstonia eutropha, Lactobacillus brevis, Lactobacillus helveticus, Leuconostoc pseudomesenteroides, Microsporum cookei* and *Moniliella acetoabatens,* among others.

These microorganisms can generally be obtained from stock strains readily available or purchasable, although they may be isolated from the natural world. It is also possible to obtain microbial strains having properties favorable for the reaction in question by causing a mutation in these microorganisms.

In cultivating these microorganisms, any of the media containing nutrient sources generally assimilable by these microorganisms can be used. For example, use can be made of ordinary media containing a carbon source or carbon sources selected from among saccharides such as glucose, sucrose and maltose, organic acids such as lactic acid, acetic acid, citric acid and propionic acid, alcohols such as ethanol and glycerol, hydrocarbons such as paraffins, fats and oils such as soybean oil and rapeseed oil, and mixtures thereof; a nitrogen source or nitrogen sources selected from among ammonium sulfate, ammonium phosphate, urea, yeast extracts, meat extracts, peptone, corn steep liquor and so forth; and, further, a nutrient source or sources selected from among inorganic salts and vitamins, among others, as appropriately formulated and mixed up. An appropriate medium can be selected from among these depending on the microorganism employed.

Generally, the cultivation of the microorganism can be carried out under ordinary conditions. For example, the cultivation is preferably carried out aerobically within a pH range of 4.0 to 9.5 and a temperature range of 20°C to 45°C for 10 to 96 hours. Generally, in reacting the microorganism with a 2-benzamidomethyl-3-oxobutyric acid ester, the culture fluid containing cells of the microorganism can be submitted as such to the reaction, and the culture broth can also be used in the form of a concentrate. In cases where a component in the culture fluid exerts an unfavorable influence on the reaction, cells or a cell treatment product obtained by subjecting the culture fluid to such treatment as centrifugation can also be used.

The cell treatment product derived from the microorganism is not particularly restricted but includes, for example, dried cells obtained by dehydration treatment using acetone or diphosphorus pentaoxide or utilizing a desiccator or electric fan, surfactant treatment products, lytic enzyme treatment products, immobilized cells and cell-free extracts obtained by cell disruption, among others. Further, an enzyme catalyzing the reduction reaction stereoselectively as purified from the culture may also be used.

### 5. Reduction reaction

In carrying out the reduction reaction, the substrate 2-(N-substituted aminomethyl)-3-oxobutyric acid esters may be added all at once at the beginning of the reaction or may be added in divided portions according to the progress of the reaction. The reaction temperature is generally 10 to 60°C, preferably 20 to 40°C, and the pH during the reaction is within the range of 2.5 to 9, preferably 5 to 9. The amount of the enzyme source in the reaction mixture can be appropriately determined according to the capability thereof to reduce the substrate. The substrate concentration in the reaction mixture is preferably 0.01 to 50% (w/v), more preferably 0.1 to 30% (w/v). The reaction is generally carried out with shaking or with stirring under aeration. The reaction time is properly determined according to the substrate concentration, enzyme source amount and other reaction conditions. Generally, the various conditions are preferably selected so that the reaction may be completed in 2 to 168 hours.

For promoting the reduction reaction, such an energy source as glucose, ethanol or isopropanol is preferably added in a proportion of 0.5 to 30% so that favorable results may be obtained. It is also possible to promote the reaction by adding such a coenzyme generally required in carrying out a biological reduction reaction as reduced nicotinamide adenine dinucleotide (hereinafter referred to as NADH for short) or reduced nicotinamide adenine dinucleotide phosphate (hereinafter referred to as NADPH for short). In this case, more specifically, they are directly added to the reaction mixture.

For promoting the reduction reaction, the reaction is preferably carried out in the simultaneous presence of an enzyme reducing NAD⁺ or NADP⁺ to the respective reduced form as well as a substrate for the reduction to give favorable results. For example, glucose dehydrogenase is caused to coexist as the enzyme for reduction to the reduced form and glucose as the substrate for reduction, or formate dehydrogenase is caused to coexist as the enzyme for reduction to the reduced from and formic acid as the substrate for reduction.

### 6. Modifications of the reduction reaction

It is also possible to produce the optically active 2-(N-substituted aminomethyl)-3-hydroxybutyric acid esters in the same manner by using, in lieu of the enzyme (reductase) catalyzing the reduction reaction according the present invention, a transformant harboring a DNA coding for that enzyme.

Further, it is possible to produce the optically active 2-(N-substituted aminomethyl)-3-hydroxybutyric acid ester in the same manner by using a transformant harboring both a DNA coding for the reductase according to the present invention and a DNA coding for a polypeptide capable of catalyzing coenzyme reproduction. In particular, when a transformant harboring both a DNA coding for the reductase according to the invention and a DNA coding for a polypeptide capable of catalyzing coenzyme reproduction is used, the optically active 3-hydroxybutyric acid esters can be produced more efficiently without the need of separately preparing and adding the enzyme for coenzyme reproduction.

The transformant harboring a DNA coding for the polypeptide according to the invention or the transformant harboring both a DNA coding for the polypeptide according to the invention and a DNA coding for a polypeptide capable of catalyzing coenzyme reproduction, not only in the form of cultured cells but also in the form of a treatment product derived therefrom, can be used in the production of the optically active 3-hydroxybutyric acid esters. The treatment product derived from the transformant, so referred to herein, is as described hereinabove.

The transformant harboring both a DNA coding for the reductase according to the invention and a DNA coding for a polypeptide capable of catalyzing coenzyme reproduction can be obtained by integrating both a DNA coding for the reductase according to the invention and a DNA coding for a polypeptide capable of catalyzing coenzyme reproduction into one and the same vector and introducing the resulting recombinant vector into host cells or by integrating these two DNAs respectively into two vectors belonging to different incompatibility groups and introducing the two recombinant vectors into host cells.

As an example of the vector resulting from integration of both a DNA coding for the reductase according to the invention and a DNA coding for a polypeptide capable of catalyzing coenzyme regeneration, there may be mentioned pNTDRG1 obtainable by introduction of the *Bacillus megaterium-*derived glucose dehydrogenase gene into the expression vector pNTDR described in WO 2004/027055. As an example of the transformant harboring both a DNA coding for the reductase according to the invention and a DNA coding for a polypeptide capable of catalyzing coenzyme reproduction, there may be mentioned *E. coli* HB101(pNTDRG1) obtainable by transforming E. coli HB101 with the vector mentioned above.

The cultivation of the transformant harboring a DNA coding for the reductase according to the invention and the cultivation of the transformant harboring both a DNA coding for the reductase according to the invention and a DNA coding for a polypeptide capable of catalyzing coenzyme reproduction can be carried out using ordinary liquid nutrient media containing a carbon source (s), a nitrogen source (s), an inorganic salt (s) and an organic nutrient(s), among others, so long as the transformant can grow therein.

Furthermore, it is also efficient to add, to the reaction mixture, such a surfactant as Triton (product of Nakalai Tesque, Inc.), Span (product of KANTO CHEMICAL CO., INC.) or Tween (product of Nakalai Tesque, Inc.). Further, for avoiding the inhibition of the reaction by the substrate and/or the reduction reaction product 3-hydroxybutyric acid esters, such an organic solvent insoluble in water as ethyl acetate, butyl acetate, isopropyl ether, toluene or hexane may be added to the reaction mixture. It is further possible to add such an organic solvent soluble in water as methanol, ethanol, acetone, tetrahydrofuran or dimethyl sulfoxide for the purpose of increasing the solubility of the substrate.

### 7. Product recovery

The method for recovering the optically active 2-(N-substituted aminomethyl)-3-hydroxybutyric acid esters formed by the reduction reaction is not particularly restricted but the optically active 2-(N-substituted aminomethyl)-3-hydroxybutyric acid esters can be readily obtained in a highly pure form by extracting the same directly from the reaction mixture or from cells or the like separated therefrom with such a solvent as ethyl acetate, toluene, tert-butyl methyl ether or hexane and, after dehydration, purifying the same by distillation or silica gel column chromatography, for instance.

### BEST MODE FOR CARRYING OUT THE INVENTION

The following examples illustrate the present invention in further detail. These examples are, however, by no means limitative of the scope of the invention. In the description which follows, "%" means "% by weight" unless otherwise specified.

In the following examples, methyl 2-benzamidomethyl-3-oxobutyrate (Examples 1 to 13), tert-butyl 2-benzamidomethyl-3-oxobutyrate (Examples 14 to 16), methyl 2-acetamidomethyl-3-oxobutyrate (Examples 17 and 18) and methyl 2-phthaloylamidomethyl-3-oxobutyrate (Examples 19 and 20) were used as examples of the reduction reaction substrate 2-(N-substituted aminomethyl)-3-oxobutyric acid esters. The reaction using a transformant is shown in Example 21.

In each example, the yield in the reduction reaction and the optical purity of the product, among others, were determined using the specified microorganism, enzyme, or enzyme and coenzyme regeneration system enzyme, among others. According to the measurement results, it was found, in each example, that the 3-hydroxybutyric acid esters having the (2S,3R) configuration can be produced with high efficiency.

### (Example 1) Reactions using microorganisms shown in Table 1

A liquid medium (pH 7) having a composition comprising 40 g of glucose, 3 g of yeast extract, 6.5 g of diammonium hydrogen phosphate, 1 g of potassium dihydrogen phosphate, 0.8 g of magnesium sulfate heptahydrate, 60 mg of zinc sulfate heptahydrate, 90 mg of iron sulfate heptahydrate, 5 mg of copper sulfate pentahydrate, 10 mg of manganese sulfate tetrahydrate and 100 mg of sodium chloride (each per liter) was distributed in 5-ml portions into large-sized test tubes, and steam-sterilized at 120°C for 20 minutes. These liquid media were respectively inoculated aseptically with the microorganisms shown in Table 1 given below (the inoculum size being one loopful), followed by 72 hours of shake culture at 30°C. After cultivation, each culture fluid was centrifuged and the thus-collected cells were suspended in 0.5 ml of 100 mM phosphate buffer (pH 6.5) containing 1% of glucose.

This cell suspension was added to a test tube containing 2.5 mg of methyl 2-benzamidomethyl-3-oxobutyrate placed therein in advance and the reaction was allowed to proceed at 30°C for 24 hours. Thereafter, 1 ml of ethyl acetate was added to each reaction mixture, followed by thorough mixing. A portion of the organic layer was analyzed using a HPLC equipped with Daicel Chemical Industries' Chiralpak AD-H (250 mm x 4.6 mm), and the yield and optical purity of the reaction product were determined. The results thus obtained are summarized in Table 1.

**Table 1**

| Microorganism | Yield (%) | Optical purity (%ee) | Diastereo⁻ selectivity (%de) | Configuration |
|---|---|---|---|---|
| *Candida kefyr* NBRC 0706 | 40 | 36.3 | 29.6 | (2S,3R) |
| *Candida oleophila* CBS 2220 | 4 | 48.6 | 100 | (2S,3R) |
| *Geotrichum eriense* NBRC 10584 | 17 | 43.8 | 55.7 | (2S,3R) |
| *Galactomyces reessii* NBRC 10823 | 23 | 17.2 | 28.6 | (28,3R) |
| *Saccharomycopsis malanga* NBRC 1710 | 4 | 96.2 | 92.2 | (2S,3R) |

### (Example 2) Reactions using microorganisms shown in Table 2

A liquid medium (pH 7) having a composition comprising 10 g of meat extract, 10 g of peptone, 5 g of yeast extract and 3 g of sodium chloride (each per liter) was distributed in 7-ml portions into large-sized test tubes and steam-sterilized at 120°C for 20 minutes. These liquid media were respectively inoculated aseptically with the microorganisms shown below in Table 2 (the inoculum size being one loopful), followed by 72 hours of shake culture at 30°C. After cultivation, each culture fluid was centrifuged and the thus-collected cells were suspended in 0.5 ml of 100 mM phosphate buffer (pH 6.5) containing 1% of glucose.

This cell suspension was added to a test tube containing 2.5 mg of methyl 2-benzamidomethyl-3-oxobutyrate placed therein in advance and the reaction was allowed to proceed at 30°C for 24 hours. Thereafter, 1 ml of ethyl acetate was added to each reaction mixture and, after thorough mixing, a portion of the organic layer was analyzed under the analysis conditions described in Example 1 and the yield and optical purity of the product were determined. The results thus obtained are summarized in Table 2.

**Table 2**

| Microorganism | Yield (%) | Optical purity (%ee) | Diastereo⁻ selectivity (%de) | Configuration |
|---|---|---|---|---|
| *Achromobacter xylosoxidans* NBRC 13495 | 3 | 75.6 | 74.8 | (2S,3R) |
| *Arthrobacter Paraffineus* ATCC 21218 | 65 | 58.5 | 74.9 | (2S.3R) |
| *Arthrobacter nicotianae* NBRC 14234 | 2 | 36.4 | 100 | (2S,3R) |
| *Bacillus amylolyticus* NBRC 15957 | 100 | 33.0 | 82.8 | (2S,3R) |
| *Bacillus circulans* ATCC 9966 | 19 | 22.4 | 13.0 | (2S,3R) |
| *Bacillus cereus* NBRC 3466 | 100 | 20.8 | 100 | (2S,3R) |
| *Bacillus badius* ATCC 14574 | 92 | 15.6 | 97.3 | (2S,3R) |
| *Bacillus sphaericus* NBRC 3525 | 39 | 14.2 | 100 | (2S,3R) |
| *Brevundimonas diminuta* NBRC 3140 | 22 | 11.1 | 49.6 | (2S,3R) |
| *Xanthomonas* sp. NBRC 3084 | 94 | 90.4 | 57.4 | (2S,3R) |
| *Xanthomonas* sp. NBRC 3085 | 100 | 99.6 | 96.9 | (2S,3R) |

### (Example 3) Reactions using microorganisms shown in Table 3

A liquid medium (pH 7) having a composition comprising 10 g of glucose, 10 g of peptone, 10 g of meat extract, 5 g of yeast extract 1 g of sodium chloride and 0.5 g of magnesium sulfate heptahydrate (each per liter) was distributed in 5-ml portions into large-sized test tubes and steam-sterilized at 120°C for 20 minutes. These liquid media were respectively inoculated aseptically with the microorganisms shown below in Table 3 (the inoculum size being one loopful), followed by 72 hours of shake culture at 28°C. After cultivation, each culture fluid was centrifuged and the thus-collected cells were suspended in 1 ml of 100 mM phosphate buffer (pH 6.5) containing 1% of glucose.

This cell suspension was added to a test tube containing 1 mg of methyl 2-benzamidomethyl-3-oxobutyrate placed therein in advance and the reaction was allowed to proceed at 30°C for 24 hours. Thereafter, 2 ml of ethyl acetate was added to each reaction mixture and, after thorough mixing, a portion of the organic layer was analyzed under the analysis conditions described in Example 1 and the yield and optical purity of the product were determined. The results thus obtained are summarized in Table 3.

**Table 3**

| Microorganism | Yield (%) | Optical purity (%ee) | Diastereo⁻ selectivity (% de) | Configuration |
|---|---|---|---|---|
| *Microsporum cookei* NBRC 7862 | 1 | 20.2 | 27.3 | (2S,3R) |
| *Moniliella acetoabatens* NBRC 9481 | 11 | 30.3 | 9.1 | (2S,3R) |

### (Example 4) Reduction reaction of methyl

### 2-benzamidomethyl-3-oxobutyrate

A liquid medium (pH 6.5) having a composition comprising 55 g (per liter) of MSR medium (product of Difco Laboratories) was distributed in 15-ml portions into large-sized test tubes and steam-sterilized at 120°C for 20 minutes. These liquid media were respectively inoculated aseptically with the microorganisms shown below in Table 4 (the inoculum size being one loopful), followed by 72 hours of stationary culture at 30°C. After cultivation, each culture fluid was centrifuged and the thus-collected cells were suspended in 1 ml of 100 mM phosphate buffer (pH 6.5) containing 1% of glucose.

This cell suspension was added to a test tube containing 1 mg of methyl 2-benzamidomethyl-3-oxobutyrate placed therein in advance and the reaction was allowed to proceed at 30°C for 24 hours. Thereafter, 2 ml of ethyl acetate was added to each reaction mixture, followed by thorough mixing. A portion of the organic layer was analyzed under the analysis conditions described in Example 1 and the yield and optical purity of the product were determined. The results thus obtained are summarized in Table 4.

**Table 4**

| Microorganism | Yield (%) | Optical purity (%ee) | Diastereo⁻ selectivity (%de) | Configuration |
|---|---|---|---|---|
| *Lactobacillus brevis* JCM 1059 | 67 | 71.7 | 92.2 | (2S,3R) |
| *Lactobacillus helveticus* JCM 1120 | 7 | 20.3 | 51.2 | (2S,3R) |
| *Leuconostoc pseudomesenteroides* JCM 9696 | 14 | 82.5 | 73.1 | (2S,3R) |

### (Example 5) Reaction using acetone-dried cells

To 1 ml of 100 mM phosphate buffer (pH 6.5), there were added 10 mg of acetone-dried cells of *Candida kefyr* NBRC 0706, 10 mg of glucose, 1 mg of glucose dehydrogenase GLUCDH "Amano 2" (product of Amano Enzyme Inc.), 0.25 mg of NAD, 0.25 mg of NADP and 2.5 mg of methyl 2-benzamidomethyl-3-oxobutyrate, and the reaction was allowed to proceed at 30°C for 24 hours. Thereafter, 2 ml of ethyl acetate was added to each reaction mixture and, after thorough mixing, a portion of the organic layer was analyzed under the analysis conditions described in Example 1; the yield was 40%. That portion of the organic layer had an optical purity of 46.8% and the diastereoselectivity was 31.8% de.

### (Example 6) Reaction using alcohol dehydrogenase

To 1 ml of 100 mM phosphate buffer (pH 6.5) were added 10 kU of *Lactobacillus brevis-*derived alcohol dehydrogenase (product of Julich Fine Chemicals), 2 equivalents of NADPH and 1 mg of methyl 2-benzamidomethyl-3-oxobutyrate, and the reaction was allowed to proceed at 30°C for 24 hours. Thereafter, each reaction mixture was extracted with 2 ml of ethyl acetate to give methyl (2S,3R)-2-benzamidomethyl-3-hydroxybutyrate in 91% yield. This product had an optical purity of 99.9% ee or above and the diastereoselectivity was 92% de.

### (Example 7) Reaction using carbonyl reductase

To 30 ml of 100 mM phosphate buffer (pH 6.5) were added 3 g of glucose, 10 kU of *Devosia riboflavina*-derived carbonyl reductase RDR (cf. WO 2004/027055), 500 mg of glucose dehydrogenase GLUCDH "Amano 2" (product of Amano Enzyme Inc.), 50 mg of NAD and 4.5 g of methyl 2-benzamidomethyl-3-oxobutyrate, and the mixture was stirred at 30°C. During the stirring, the pH of the reaction mixture was maintained at 6.5 with 6 N NaOH. After 24 hours of reaction, the reaction mixture was extracted with three 45-ml portions of ethyl acetate, and the organic layers obtained were combined and dried over anhydrous sodium sulfate. The sodium sulfate was filtered off, the organic solvent was distilled off under reduced pressure, and the residue was purified by silica gel column chromatography to give 4.4 g of methyl (2S,3R)-2-benzamidomethyl-3-hydroxybutanoate. This product had an optical purity of 99% ee or above, and the diastereoselectivity was 89.8% de.
[α]²⁵_{D} +22.88° (c = 0.9, ethyl acetate)
¹H-NMR (400 MHz, CDCl₃, δ ppm) : 7.8-7.7 (m, 2H), 7.6-7.5 (m, 1H), 7.5-7.4 (m, 2H), 6.9 (br. s, 1H), 4.2-4.0 (m, 1H), 4.0-3.9 (m, 1H), 3.7 (s, 3H), 3.6-3.5 (m, 1H), 2.8 (m, 1H), 1.2 (d, 3H)

### (Example 8) Reaction using carbonyl reductase

To 1 ml of 100 mM phosphate buffer (pH 6.5) were added 50 mg of glucose, 10 kU of *Candida maris*-derived carbonyl reductase FPDH (cf. WO 01/05996), 1 mg of glucose dehydrogenase GLUCDH "Amano 2" (product of Amano Enzyme Inc.), 0.25 mg of NAD and 5 mg of methyl 2-benzamidomethyl-3-oxobutyrate, and the mixture was shaken at 30°C for 24 hours. Thereafter, the reaction mixture was extracted with 2 ml of ethyl acetate to give methyl (2S,3R)-2-benzamidomethyl-3-hydroxybutanoate in 99% yield. This product had an optical purity of 94% ee, and the diastereoselectivity was 89.3% de.
¹H-NMR (400 MHz, CDCl₃, δ ppm): 7.8-7.7 (m, 2H), 7.6-7.5 (m, 1H), 7.5-7.4 (m, 2H), 6.9 (br. s, 1H), 4.2-4.0 (m, 1H), 4.0-3.9 (m, 1H), 3.7 (s, 3H), 3.6-3.5 (m, 1H), 2.8 (m, 1H), 1.2 (d, 3H)

### (Example 9) Reaction using acetoacetyl-CoA reductase

To 1 ml of 100 mM phosphate buffer (pH 6.5) were added 50 mg of glucose, 10 kU of *Ralstonia eutropha*-derived acetoacetyl-CoA reductase RRE (cf. WO 2005/044973), 1 mg of glucose dehydrogenase GLUCDH "Amano 2" (product of Amana Enzyme Inc.), 0.25 mg of NAD and 5 mg of methyl 2-benzamidomethyl-3-oxobutyrate, and the mixture was shaken at 30°C. After 24 hours of reaction, the reaction mixture was extracted with 2 ml of ethyl acetate to give methyl (2S,3R)-2-benzamidomethyl-3-hydroxybutanoate in 54% yield. This product had an optical purity of 84.8% ee, and the diastereoselectivity was 60% de.

### (Example 10) Reaction using acetoacetyl-CoA reductase

To 1 ml of 100 mM phosphate buffer (pH 6.5) were added 50 mg of glucose, 10 kU of *Achromobacter denitrificans*-derived acetoacetyl-CoA reductase RAX (cf. WO 2005/044973), 1 mg of glucose dehydrogenase GLUCDH "Amano 2" (product of Amano Enzyme Inc.), 0.25 mg of NAD and 5 mg of methyl 2-benzamidomethyl-3-oxobutyrate, and the mixture was shaken at 30°C. After 24 hours of reaction, the reaction mixture was extracted with 2 ml of ethyl acetate to give methyl (2S,3R)-2-benzamidomethyl-3-hydroxybutanoate in 57% yield. This product had an optical purity of 59.7% ee, and the diastereoselectivity was 49.5% de.

### (Example 11) Reduction reaction of methyl 2-benzamidomethyl-3-oxybutyrate

A liquid medium (pH 7) having a composition comprising 10 g of meat extract, 10 g of peptone, 5 g of yeast extract and 3 g of sodium chloride (each per liter) was distributed in 7-ml portions into large-sized test tubes and steam-sterilized at 120°C for 20 minutes. These liquid media were respectively inoculated aseptically with the microorganisms shown below in Table 5 (the inoculum size being one loopful), followed by 72 hours of shake culture at 30°C. Thereafter, each culture fluid was centrifuged and the thus-collected cells were suspended in 0.5 ml of 100 mM phosphate buffer (pH 6.5) containing 1% of glucose.

This cell suspension was added to a test tube containing 0.5 mg of ethyl 2-benzamidomethyl-3-oxobutyrate placed therein in advance and the reaction was allowed to proceed at 30°C for 24 hours. Thereafter, 1 ml of ethyl acetate was added to each reaction mixture and, after thorough mixing, a portion of the organic layer was analyzed using a HPLC equipped with Daicel Chemical Industries' Chiralpak AD-H (250 mm x 4.6 mm), and the yield and optical purity of the reaction product were determined. The results thus obtained are summarized in Table 5.

**Table 5**

| Microorganism | Yield (%) | Optical purity (%ee) | Diastereo⁻ selectivity (de) | Configuration |
|---|---|---|---|---|
| *Achromobacter xylosoxidans* NBRC 13495 | 10 | 36.3 | 20.6 | (2S,3R) |
| *Arthrobacter paraffineus* ATCC 21218 | 63 | 100.0 | 95.3 | (2S,3R) |
| *Bacillus amylolyticus* NBRC 15957 | 100 | 45.5 | 100 | (2S,3R) |
| *Bacillus circulans* ATCC 9966 | 2 | 17.0 | 8.1 | (2S,3R) |
| *Bacillus cereus* NBRC 3466 | 39 | 13.9 | 100 | (2S,3R) |
| *Bacillus badius* ATCC 1.4574 | 2 | 0.3 | 18.9 | (2S,3R) |
| *Brevundimonas diminuta* NBRC 3140 | 1 | 100.0 | 13.0 | (2S,3R) |
| *Xanthomonas* sp. NBRC 3084 | 1 | 100.0 | 100 | (2S,3R) |
| *Xanthomonas* sp. NBRC 3085 | 1 | 100.0 | 42.5 | (2S,3R) |

### (Example 12) Reaction using carbonyl reductase

To 1 ml of 100 mM phosphate buffer (pH 6.5) were added 50 mg of glucose, 10 kU of *Devosia riboflavina*-derived carbonyl reductase RDR (cf. WO 2004/027055), 1 mg of glucose dehydrogenase GLUCDH "Amano 2" (product of Amano Enzyme Inc.), 0.25 mg of NAD and 5 mg of ethyl 2-benzamidomethyl-3-oxobutyrate, and the mixture was shaken at 30°C. After 24 hours of reaction, the reaction mixture was extracted with 2 ml of ethyl acetate to give ethyl (2S,3R)-2-benzamidomethyl-3-hydroxybutanoate in 100% yield. This product had an optical purity of 96% ee, and the diastereoselectivity was 91% de.
¹H-NMR (400 MHz, CDCl₃, δ ppm): 7.8-7.3 (m, 5H), 6.9 (br. s, 1H), 4.2-4.0 (m, 3H), 4.0-3.9 (m, 2H), 2.4 (s, 3H), 1.2 (t, 3H)

### (Example 13) Reaction using carbonyl reductase

To 1 ml of 100 mM phosphate buffer (pH 6.5) were added 50 mg of glucose, 10 kU of *Candida maris*-derived carbonyl reductase FPDH (cf. WO 01/05996), 1 mg of glucose dehydrogenase GLUCDH "Amano 2" (product of Amano Enzyme Inc.), 0.25 mg of NAD and 5 mg of ethyl 2-benzamidomethyl-3-oxobutyrate, and the mixture was shaken at 30°C. After 24 hours of reaction, the reaction mixture was extracted with 2 ml of ethyl acetate to give ethyl (2S,3R)-2-benzamidomethyl-3-hydroxybutanoate in 61% yield. This product had an optical purity of 71.8% ee, and the diastereoselectivity was 71.4% de.

### (Example 14) Reaction using microorganisms shown in Table 6

A liquid medium (pH 7) having a composition comprising 10 g of meat extract, 10 g of peptone, 5 g of yeast extract and 3 g of sodium chloride (each per liter) was distributed in 7-ml portions into large-sized test tubes and steam-sterilized at 120°C for 20 minutes. These liquid media were respectively inoculated aseptically with the microorganisms shown below in Table 6 (the inoculum size being one loopful), followed by 72 hours of shake culture at 30°C. Thereafter, each culture fluid was centrifuged and the thus-collected cells were suspended in 0.5 ml of 100 mM phosphate buffer (pH 6.5) containing 1% of glucose. This cell suspension was added to a test tube containing 0.5 mg of tert-butyl 2-benzamidomethyl-3-oxobutyrate placed therein in advance and the reaction was allowed to proceed at 30°C for 24 hours. Thereafter, 1 ml of ethyl acetate was added to each reaction mixture and, after thorough mixing, a portion of the organic layer was analyzed using a HPLC equipped with Daicel Chemical Industries' Chiralpak AD-H (250 mm x 4.6 mm), and the yield and optical purity of the reaction product were determined. The results thus obtained are summarized in Table 6.

**Table 6**

| Microorganism | Yield (%) | Optical purity (%ee) | Diastereo⁻ selectivity (%de) | Configuration |
|---|---|---|---|---|
| *Xanthomonas* sp. NBRC 3084 | 1 | 21.5 | 88.9 | (2S,3R) |
| *Xanthomonas* sp. NBRC 3085 | 1 | 16.9 | 100 | (2S,3R) |

### (Example 15) Reaction using carbonyl reductase

To 1 ml of 100 mM phosphate buffer (pH 6.5) were added 50 mg of glucose, 10 kU of *Devosia riboflavina*-derived carbonyl reductase RDR (cf. WO 2004/027055), 1 mg of glucose dehydrogenase GLUCDH "Amano 2" (product of Amano Enzyme Inc.), 0.25 mg of NAD and 5 mg of tert-butyl 2-benzamidomethyl-3-oxobutyrate, and the mixture was shaken at 30°C. After 24 hours of reaction, the reaction mixture was extracted with 2 ml of ethyl acetate to give tert-butyl (2S,3R)-2-benzamidomethyl-3-hydroxybutanoate in 88% yield. This product had an optical purity of 99.9% ee or above, and the diastereoselectivity was 95.3% de.
¹H-NMR (400 MHz, CDCl₃, δ ppm): 7.8-7.3 (m, 5H), 6.9 (br. s, 1H), 4.0-3.9 (m, 4H), 2.4 (s, 3H), 1.2 (s, 9H)

### (Example 16) Reaction using carbonyl reductase

To 1 ml of 100 mM phosphate buffer (pH 6.5) were added 50 mg of glucose, 10 kU of *Candida maris*-derived carbonyl reductase FPDH (cf. WO 01/05996), 1 mg of glucose dehydrogenase GLUCDH "Amano 2" (product of Amano Enzyme Inc.), 0.25 mg of NAD and 5 mg of tert-butyl 2-benzamidomethyl-3-oxobutyrate, and the mixture was shaken at 30°C. After 24 hours of reaction, the reaction mixture was extracted with 2 ml of ethyl acetate to give tert-butyl (2S,3R)-2-benzamidomethyl-3-hydroxybutanoate in 15% yield. This product had an optical purity of 91.4% ee, and the diastereoselectivity was 77.7% de.

### (Example 17) Reaction using carbonyl reductase

To 1 ml of 100 mM phosphate buffer (pH 6.5) were added 50 mg of glucose, 10 kU of *Devosia riboflavina*-derived carbonyl reductase RDR (cf. WO 2004/027055), 1 mg of glucose dehydrogenase GLUCDH "Amano 2" (product of Amano Enzyme Inc.), 0.25 mg of NAD and 5 mg of methyl 2-acetamidomethyl-3-oxobutyrate, and the mixture was shaken at 30°C. After 24 hours of reaction, the reaction mixture was extracted with 2 ml of ethyl acetate to give methyl (2S,3R)-2-acetamidomethyl-3-hydroxybutanoate in 59% yield. This product had an optical purity of 97% ee as determined using a HPLC equipped with Daicel Chemical Industries' Chiralpak AD-H (250 mm x 4.6 mm), and the diastereoselectivity was 82.3% de. ¹H-NMR (400 MHz, CDCl₃, δ ppm) : 6.2 (br. s, 1H), 4.0-3.7 (m, 2H), 3.6 (s, 1H), 3.4-3.3 (m, 1H), 2.7-2.5 (m, 1H), 2.2 (s, 3H), 1.2 (d, 3H)

### (Example 18) Reaction using carbonyl reductase

To 1 ml of 100 mM phosphate buffer (pH 6.5) were added 50 mg of glucose, 10 kU of *Candida marts*-derived carbonyl reductase FPDH (cf. WO 01/05996), 1 mg of glucose dehydrogenase GLUCDH "Amano 2" (product of Amano Enzyme Inc.), 0.25 mg of NAD and 5 mg of methyl 2-acetamidomethyl-3-oxobutyrate, and the mixture was shaken at 30°C. After 24 hours of reaction, the reaction mixture was extracted with 2 ml of ethyl acetate to give methyl (2S,3R)-2-acetamidomethyl-3-hydroxybutanoate in 8% yield. This product had an optical purity of 96.2% ee, and the diastereoselectivity was 80.3% de.

### (Example 19) Reaction using carbonyl reductase

To 1 ml of 100 mM phosphate buffer (pH 6.5) were added 50 mg of glucose, 10 kU of *Devosia riboflavina-derived* carbonyl reductase RDR (cf. WO 2004/027055), 1 mg of glucose dehydrogenase GLUCDH "Amano 2" (product of Amano Enzyme Inc.), 0.25 mg of NAD and 5 mg of methyl 2-phthaloylamidomethyl-3-oxobutyrate, and the mixture was shaken at 30°C. After 24 hours of reaction, the reaction mixture was extracted with 2 ml of ethyl acetate to give methyl (2S,3R)-2-phthaloylamidomethyl-3-hydroxybutanoate in 98% yield. This product had an optical purity of 99.9% ee or above as determined using a HPLC equipped with Daicel Chemical Industries' Chiralpak AD-H (250 mm x 4.6 mm), and the diastereoselectivity was 62.2% de.
¹H-NMR (400 MHz, CDCl₃, δ ppm): 7.9-7.7 (m, 4H), 4.2-3.9 (m, 3H), 3.7 (s, 3H), 2.7-2.6 (m, 1H), 1.2 (d, 3H)

### (Example 20) Reaction using carbonyl reductase

To 1 ml of 100 mM phosphate buffer (pH 6.5) were added 50 mg of glucose, 10 kU of *Candida maris*-derived carbonyl reductase FPDH (cf. WO 01/05996), 1 mg of glucose dehydrogenase GLUCDH "Amano 2" (product of Amano Enzyme Inc.), 0.25 mg of NAD and 5 mg of methyl 2-phthaloylamidomethyl-3-oxobutyrate, and the mixture was shaken at 30°C. After 24 hours of reaction, the reaction mixture was extracted with 2 ml of ethyl acetate to give methyl (2S,3R)-2-phthaloylamidomethyl-3-hydroxybutanoate in 21% yield. This product had an optical purity of 99.9% ee or above and the diastereoselectivity was 72.4% de.

### (Example 21) Reaction using transformant

*E. coli* HB101(pNTDRG1) (FERM BP-08458; cf. WO 2004/027055) was cultured in 2xYT medium containing 120 µg/ml of ampicillin. To 30 ml of the thus-obtained culture fluid were added 2 g of glucose, 50 mg of NAD and 3 g of methyl 2-benzamidomethyl-3-oxobutyrate, and the mixture was stirred at 30°C while the pH of the reaction mixture was maintained at 6.5 with 6 N NaOH. After 24 hours of reaction, the reaction mixture was extracted with three 30-ml portions of ethyl acetate, and the organic layers obtained were combined and dried over anhydrous sodium sulfate. The sodium sulfate was filtered off, the organic solvent was distilled off under reduced pressure, and the residue was purified by silica gel chromatography to give 2.8 g of methyl (2S,3R)-2-benzamidomethyl-3-hydroxybutanoate. This product had an optical purity of 99% ee or above, and the diastereoselectivity was 89.8% de.

## Claims

1. A method for producing optically active 2-(N-substituted aminomethyl)-3-hydroxybutyric acid esters represented by the general formula (5): 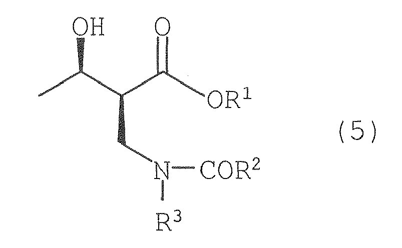 (wherein R¹ represents a lower alkyl group, which may optionally be substituted, an allyl group, an aryl group, which may optionally be substituted, or an aralkyl group, which may optionally be substituted and, as for R² and R³,
1) R³ is a hydrogen atom and R² represents a lower alkyl group, which may optionally be substituted, an alkoxy group, which may optionally be substituted, an aryl group, which may optionally be substituted, or an aralkyloxy group, which may optionally be substituted, or
2) R³ and -COR² together represent a phthaloyl group):,
wherein a 2-(N-substituted aminomethyl)-3-oxobutyric acid ester represented by the general formula (6): 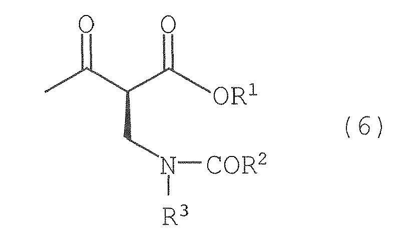 (wherein R¹, R² and R³ are as defined above): is treated with an enzyme source capable of stereoselectively reducing said ester to the corresponding optically active 3-hydroxybutyric acid ester having the (2S,3R) configuration.

2. The method according to Claim 1
wherein a compound represented by the general formula (1) : 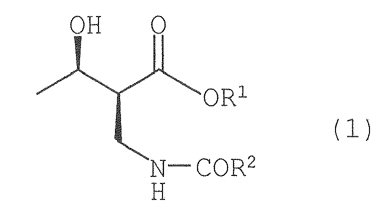 (wherein R¹ is as defined above, R² represents a lower alkyl group, which may optionally be substituted, a lower alkoxy group, which may optionally be substituted, an aryl group, which may optionally be substituted, or an aralkyloxy group, which may optionally be substituted): is produced, as the optically active 2-(N-substituted aminomethyl)-3-hydroxybutyric acid ester represented by the formula (5), using a compound represented by the general formula (2): 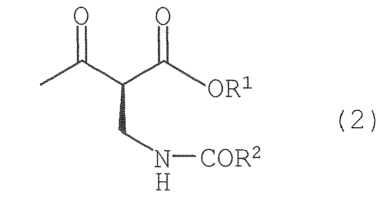 (wherein R¹ and R² are as defined above) : as the 2- (N-substituted aminomethyl)-3-oxobutyric acid ester represented by the formula (6).

3. The method according to Claim 1
wherein a compound represented by the general formula (3): 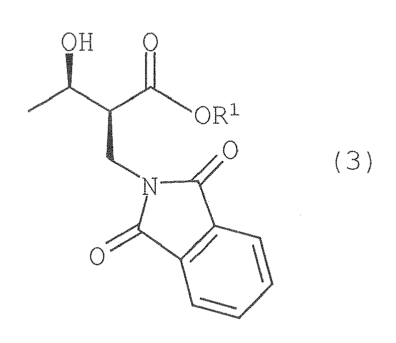 (wherein R¹ is as defined above) : is produced, as the optically active 2-(N-substituted aminomethyl)-3-hydroxybutyric acid ester represented by the formula (5), using a compound represented by the general formula (4): 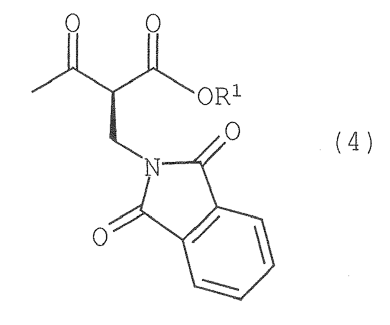 (wherein R¹ is as defined above): as the 2-(N-substituted aminomethyl)-3-oxobutyric acid ester represented by the formula (6).

4. The method according to any one of Claims 1 to 3
wherein the enzyme source is an enzyme derived from the microorganism selected from the group consisting of microorganisms belonging to the genera *Candida, Geotrichum, Galactomyces, Saccharomycopsis, Achromobacter, Arthrobacter, Bacillus, Brevundimonas, Xanthomonas, Devosia, Ralstonia, Lactobacillus, Letaconostoc, Microsporum* and *Moniliella.*

5. The method according to any one of Claims 1 to 3 wherein the enzyme source is an enzyme derived from the microorganism selected from the group consisting of microorganisms of such species as *Candida kefyr, Candida oleaphila, Candida maris, Geotrichum eriense, Galactomyces reessii, Saccharomycopsis malanga, Achromobacter xylosoxidans, Achramabacter denitrificans, Arthrobacter paraffineus, Arthrobacter nicotianae, Bacillus amylolytlcus, Bacillus circulans, Bacillus cereus, Bacillus badius, Bacillus sphaericus, Brevundimonas diminuta, Xanthomonas* sp., *Devosia riboflavina, Ralstonia eutropha, Lactobacillus brevis, Lactobacillus helveticus, Leuconostoc pseudomesenteroides, Microsporum cookei* and *Moniliella acetoabatens.*

6. The method according to any one of Claims 1 to 3
wherein R¹ is a methyl group, an ethyl group, a propyl group, or an n-butyl group.

7. The method according to any one of Claims 1 to 3
wherein R¹ is a methyl group.

8. The method according to Claim 1 or 2
wherein R² is a phenyl group, a p-nitrophenyl group, or a p-chlorophenyl group.

9. The method according to Claim 1 or 2
wherein R² is a phenyl group.

10. The method according to Claim 1 or 2
wherein R¹ is a methyl group, and R² is a phenyl group.

11. The method according to any one of Claims 1 to 10
wherein an enzyme reducing either or both of oxidized nicotinamide adenine dinucleotide (NAD⁺) and oxidized nicotinamide adenine dinucleotide phosphate (NADP⁺) to the respective reduced form, and a substrate for the reduction coexist.
